(19) Europäisches Patentamt
European Patent Office
Office européen des brevets

(11) Publication number: **0 463 173 B1**

(12) **EUROPEAN PATENT SPECIFICATION**

(45) Date of publication of patent specification: **07.12.94**

(51) Int. Cl.5: **A61K 7/06**, A61K 31/47, A61K 31/395

(21) Application number: **91901535.4**

(22) Date of filing: **26.12.90**

(86) International application number: **PCT/JP90/01705**

(87) International publication number: **WO 91/10419 (25.07.91 91/17)**

# (54) HAIR TONIC AND DRESSING COMPOSITION.

(30) Priority: **08.01.90 JP 1315/90**

(43) Date of publication of application: **02.01.92 Bulletin 92/01**

(45) Publication of the grant of the patent: **07.12.94 Bulletin 94/49**

(84) Designated Contracting States: **CH DE DK ES FR GB IT LI NL SE**

(56) References cited:
**FR-A- 2 434 809**
**JP-A- 5 645 414**
**JP-A- 5 646 810**
**JP-A- 5 649 378**

**Copy of Abstract JP-A-5645414**

**Copy of Abstract JP-A-5646810**

(73) Proprietor: **OTSUKA PHARMACEUTICAL CO., LTD.**
**9, Kandatsukasa-cho 2-chome**
**Chiyoda-ku Tokyo 101 (JP)**

(72) Inventor: **OGITA, Zenichi**
**2556-4, Gofuku-suehiro-cho**
**Toyama-shi Toyama 930 (JP)**
Inventor: **TSUTSUI, Masahiro**
**1-32, Fukushima 2-chome**
**Tokushima-shi Tokushima 770 (JP)**
Inventor: **KIMURA, Yukio**
**8-29, Sumiyoshi 3-chome**
**Tokushima-shi Tokushima 770 (JP)**
Inventor: **ISHIZUE, Yoshihiro**
**200-3, Takase**
**Kamiita-cho**
**Itano-gun Tokushima 771-13 (JP)**
Inventor: **ISHIDA, Kozo**
**48-12, Tainohama-Aza-Nishinosu**
**Kitajima-cho Itano-gun Tokushima 771-02 (JP)**

EP 0 463 173 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Arzneimittel Forschung Drug Research, Vol. 35, No. 7A, (1985), Shintani S. et al. (General Pharmacological Properties of cilostazol, a New Antithrombotic Drug) p. 1163-1172**

Inventor: **FUKUNAGA, Yuichiro**
**463-10, Kagasuno**
**Kawauchi-cho**
**Tokushima-shi Tokushima 771-01 (JP)**

(74) Representative: **von Kreisler, Alek, Dipl.-Chem. et al**
**Patentanwälte**
**von Kreisler-Selting-Werner**
**Postfach 10 22 41**
**D-50462 Köln (DE)**

## Description

### Technical Field of the Invention

The present invention relates to a use of a carbostyril derivative having the general formula (1):

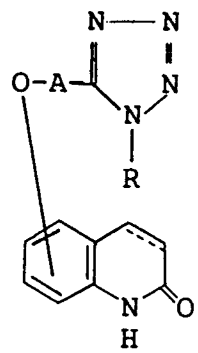

wherein A is a lower alkylene group, R is a cycloalkyl group, the bond between the 3-position and the 4-position of the carbostyril residue represents a single bond or a double bond, or a salt thereof, preferably 6-[4-(1-cyclohexyl-1,2,3,4-tetrazol-5-yl)butoxy]-3,4-dihydrocarbostyril (development No. OPC-13013; generic name: Cilostazol; trade name: Pletaal) or a salt thereof for preparing a composition for hair-growing and hair-dressing.

### Technical Background

The above carbostyril derivative having the general formula (1) and a process for preparation thereof is described in Japanese Patent Second publication (Kokoku) No. 20235/1988 and is also known to be useful as a platelet agglutination inhibitor.

On the other hand, there have been many studies as to an active ingredient to be used for hair-growing, hair-dressing and the like, but there still exists a need for developing a novel active ingredient.

### Disclosure of the Invention

The present inventors have extensively studied in order to develop a novel active ingredient for hair-growing and hair-dressing, and have found that the above carbostyril derivative of the general formula (1), especially 6-[4-(1-cyclohexyl-1,2,3,4-tetrazol-5-yl)butoxy]-3,4-dihydrocarbostyril or a salt thereof shows excellent hair grow-promoting and hair-dressing activities and hence is useful as an active ingredient for hair-growing and hair-dressing, and then have completed the present invention. That is, the present invention provides the use of the above carbostyril derivative of the general formula (1) for preparing a comsition for hair-growing and hair-dressing. Such composition is useful as an agent for preventing fall-out of hair and promoting hair-growing, an agent for treating alopecia and leukoplakia, and as a hair-dressing agent for keeping moisture or lustering hair.

The lower alkylene group in the above general formula (1) includes an alkylene group having 1 to 6 carbon atoms such as methylene, ethylene, trimethylene, tetramethylene, pentamethylene, hexamethylene, and 2-methyltrimethylene, tetramethylene being preferred. The cycloalkyl group includes a cycloalkyl group having 3 to 8 carbon atoms such as cyclopropyl, cyclobutyl, cyclopentyl, cyclohexyl, cycloheptyl and cycloocytyl, cyclohexyl being prefered.

For use of the composition of the present invention as the hair grow-promoting agent, the carbostyril derivative of the above general formula (1) or a salt thereof is formulated into a pharmaceutical preparation. Such a preparation is made using a conventional diluent or carrier such as a filler, a bulking agent, a binder, a humectant, a disintegrator, a surfactant and a lubricant. Various forms of such pharmaceutical preparation can be selected depending on a kind of treatment and representative examples are tablets, pills, powders, liquids, suspensions, emulsions, granules, capsules, depositories, injections (liquids, suspensions), and external preparations such as liquid paints, lotions, aerosols, liniments, ointments and cataplasms.

For preparation of tablets, a wide variety of carriers known in the art can be used. Such carrier includes, for example, a vehicle such as lactose, sucrose, sodium chloride, glucose, urea, starch, calcium carbonate, kaolin, crystalline cellulose, silicic acid; a binder such as water, ethanol, propanol, simple syrup, a glucose solution, a starch solution, a gelatin solution, carboxymethylcellulose, ceramic, methylcellulose, calcium phosphate, polyvinyl pyrrolidone; a disintegrator such as dried starch, sodium alginate, agar powder, laminaran powder, sodium hydrogen carbonata, calcium carbonate, polyoxyethylene sorbitan fatty acid esters, sodium lauryl sulfate, stearic acid monoglyceride, starch, lactose; a disintegration inhibitor such as sucrose, stearin, cacao butter, hydrogenated oil; an absorption accelerator such as a quarternary ammonium salt, sodium lauryl sulfate; a humectant such as glycerol, starch; an absorbant such as starch, lactose, kaolin, bentonite, colloidal silicic acid; a lubricant such as purified tale, stearic acid salt, boric acid powder, polyethylene glycol. Furthermore, the tablets may be in the form of tablets with a conventional coating as required, including for example, tablets with a sugar-coating, tablets with a gelatin-coating, tablets with an enteric coating, tablets with a film-coating, or double-layered tablets or multi-layered tablets.

For preparation of pills, a wide variety of carriers known in the art can be used. Such carrier includes, for example, a vehicle such as glucose, lactose, starch, cacao butter, hydrogenated vegetable oil, kaolin, talc; a binder such as powdered acacia, powdered tragacanth, gelatin, ethanol; a disintegrator such as laminaran, agar. For preparation of depositories, a wide variety of carriers known in the art can be used. Such carrier includes, for example, polyethylene glycol, cacao butter, a higher alcohol, esters of a higher alcohol, gelatin, semi-synthesized glyceride. In case of the preparation of injections, preferably liquids and suspensions are sterilized and is made isotonic with blood. For preparation of these liquids, emulsions and suspensions, any material used in the art as a diluent can be employed, including, for example, water, ethyl alcohol, propylene glycol, ethoxyisostearyl alcohol, polyoxyisostearyl alcohol, polyoxyethylene sorbitan fatty acid ester. In this case, the medicament may comprise sodium chloride, glucose or glycerol in an amout sufficient for preparing an isotonic solution. The medicament may further comprise conventional solubilizing agents, buffering agents, anesthetic agents, and if necessary, other additives such as coloring agents, preservatives, perfumes, flavors, sweetening agents and other medicaments.

An amount of the carbostyril derivative (1) or a salt thereof to be used in the hair grow-promoting agent of the present invention is not particularly limited but selected from a wide range. It is usually in the range of 1 to 70 % by weight, preferably from 5 to 50 % by weight based on a total weight of the composition.

An administration route of the hair grow-promoting agent of the present invention is not particularly limited and a suitable administration route may be selected depending on a dosage form, age, sex or other conditions of patient and severity of the disease. For example, when the medicament is in the form of tablets, pills, liquids, suspensions, emulsions, granules and capsules, it is orally administered. In case of injections, it is administered intravenously either alone or in admixture with a conventional ancillary solution such as glucose, amino acid, or if necessary, it is solely administered intramuscularly, intradermally, subcutaneously or intraperitoneally. Suppositories is administered rectally.

The dosage of the hair grow-promoting agent of the present invention is suitably determined depending on a usage, age, sex or other conditions of patient, severity of the disease, and may usually range from 0.6 to 50 mg/kg body weight of the carbostyril derivative (1) or a salt thereof per day. The dosage unit may contain 10 to 1000 mg of the active ingredient.

When the hair grow-promoting agent of the present invention is used in the form of external preparation, it is applied to the affected part by spraying, spreading, once to several times a day in an amount sufficient for covering a whole part of the affected part.

When the composition of the present invention is used for hair-dressing, it is generally used in the form of a cosmetics for hair.

The cosmetics of the present invention can be prepared in various forms in the same manner as the usual cosmetics except that the carbostyril derivative of the general formula (1) or a salt thereof is employed as an active ingredient. The cosmetics of the present invention includes, for example, a cosmetics for hair to be applied to head skin or hair in various forms including shampoo, rinse, hair liquid, set lotion, hair tonic. These cosmetics in various forms can be prepared in a conventional manner wherein they also contain a variety of known base material such as alcohols (e.g. ethanol, isopropyl alcohol, benzyl alcohol, water; and if necessary, various kinds of an additive including a flavoring agent such as floral flavor, citrus fruits flavor, cinnamonic flavor; an antioxidant such as dl-$\alpha$-tocopherol, butyl hydroxyanisol (BHA), chlorobutanol; a surfactant such as fatty acid glycerides (e.g. ethylene glycol monocaprylate, propylene glycol monocaprylate, propylene glycol dicaprate, glyceryl monocaprylate, glyceryl monocaprate), polyoxyalkylene sorbitan fatty acid esters (e.g. polyoxyethylene sorbitan monostearate, polyoxyethylene sorbitan tristearate); a preservative such as parabens (e.g. methylparaben, ethylparaben, butylparaben), phenols (e.g. hexachlorophenol, isopropylmethylphenol); a humectant such as glycerol, D-sorbitol, triacetin; a solubilizing

agent such as propylene carbonate, propylene glycol.

An amount of the base materials and the additives contained in the cosmetics for hair of the present invention is not particularly limited and suitably selected from a wide range. For example, the amount of the cosmetics base material may generally range from 40 to 80 w/v%, preferably from 50 to 70 w/v% based on the whole amount of the cosmetics for hair of the present invention. The amount of the antioxidant may generally range from 0.05 to 1 w/v%, preferably from 0.1 to 0.5 w/v% based on the whole amount of the cosmetics for hair. The amount of the humectant may generally range from 0.1 to 15 w/v%, preferably from 0.5 to 10 w/v% based on the whole amount of the cosmetics for hair. The amount of the solubilizing agent may generally range from 0.5 to 30 w/v%, preferaly from 1.0 to 25 w/v% based on the whole amount of the cosmetics for hair. The amount of the surfactant may generally range from 0.5 to 10 w/v%, preferably from 1.0 to 5.0 w/v%.

The amount of the carbostyril derivative of the general formula (1) or a salt thereof contained in the cosmetics of the present invention may be suitably determined depending on the form of the cosmetics prepared, a desired effect and may generally range from 0.0001 to 30 % by weight, preferably from 0.0001 to 1 % by weight in the whole composition.

## Best Mode for Practicing the Invention

The composition for hair-growing and hair-dressing of the present invention is hereinafter explained in more detail by means of the following Preparations and Pharmacological Experiment.

### Preparation 1

| | |
|---|---|
| 6-[4-(1-Cyclohexyl-1,2,3,4-tetrazol-5-yl)-butoxy]-3,4-dihydrocarbostyril | 150 g |
| Avicel (trade name, manufactured by Asahi Chemical Industry Co., Ltd.) | 40 g |
| Corn starch | 30 g |
| Magnesium stearate | 2 g |
| Hydroxypropylmethylcellulose | 10 g |
| Polyethylene glycol-6000 | 3 g |
| Castor oil | 40 g |
| Methanol | 40 g |

The active compound of the present invention, Avicel, corn starch and magensium stearate are mixed together and ground and then the mixture is tabletted using a pounder (R 10 mm) for sugar-coating. The obtained tablets are then coated with a film-coating agent comprising hydroxypropylmethylcellulose, polyethylene glycol-6000, castor oil and methanol to prepare film-coated tablets.

### Preparation 2

| | |
|---|---|
| 6-[4-(1-cyclohexyl-1,2,3,4-tetrazol-5-yl)-butoxy]-3,4-dihydrocarbostyril | 150 g |
| Citric acid | 1.0 g |
| Lactose | 33.5 g |
| Dicalcium phosphate | 70.0 g |
| Pullonic F-68 | 30.0 g |
| Sodium lauryl sulfate | 15.0 g |
| Polyvinylpyrrolidone | 15.0 g |
| Polyethylene glycol (Carbowax 1500) | 4.5 g |
| Polyethylene glycol (Carbowax 6000) | 45.0 g |
| Corn starch | 30.0 g |
| Dry sodium lauryl sulfate | 3.0 g |
| Dry magnesium stearate | 3.0 g |
| Ethanol | q.s. |

The active compound of the present invention, citric acid, lactose, dicalcium phosphate, Pullonic F-68 and sodium lauryl sulfate are mixed together.

The above mixture is sieved with No. 60 screen and wet-granulated with an alcoholic solution containing polyvinylpyrrolidone, Carbowax 1500 and 6000. If necessary, alcohol is added to the powder to produce a paste-like mass. Corn starch is added and mixing is continued until uniform particles are obtained. After passing through No. 10 screen, the obtained particles are put on a tray and dried in an oven at 100 °C for 12 to 14 hours. After the dried particles are sieved through No. 16 screen and thereto are added dry sodium lauryl sulfate and dry magnesium stearate, the resultant is mixed and the mixture is compressed into a desired form with a tabletting machine.

The core tablets thus prepared are varnished and dusted with talc in order to guard from wetting. Under-coating is applied to the core tablets. In order to administer the tablets orally, the core tablets are varnished sufficient times. In order to give completely round shape and smooth surface to the tablets, further undercoating and coating with lubricant are applied thereto. The tablets are further coated with a coloring coating material until the desired colored tablets are obtained. After drying, the coated tablets are polished to obtain the desired tablets having uniform gloss.

Preparation 3

| | |
|---|---|
| 6-[4-(1-Cyclohexyl-1,2,3,4-tetrazol-5-yl)-butoxy]-3,4-dihydrocarbostyril | 5 g |
| Polyethylene glycol (molecular weight: 4000) | 0.3 g |
| Sodium chloride | 0.9 g |
| Polyoxyethylene sorbitan monooleate | 0.4 g |
| Sodium metabisulfate | 0.1 g |
| Methylparaben | 0.18 g |
| Propylparaben | 0.02 g |
| Distilled water for injection | 10.0 ml |

The above parabens, sodium metabisulfate and sodium chloride are dissolved in distilled water of about half volume of the above with stirring at 80 °C. The obtained solution is cooled to 40 °C and then polyethylene glycol and polyoxyethylene sorbitan monooleate are dissolved in the solution. To the resultant solution is then added distilled water for injection to adjust to the desired volume, and the solution is sterilized by filtering with an appropriate filter paper to give an injection preparation.

Preparation 4

| | |
|---|---|
| 6-[4-(1-Cyclohexyl-1,2,3,4-tetrazol-5-yl)-butoxy]-3,4-dihydrocarbostyril | 0.5 g |
| Ethanol | 66.0 g (75 ml) |
| Propylene carbonate | 10.0 g |
| Glycerol | 1.5 g |
| Water | q.s. |
| Total volume | 100 ml |

Propylene carbonate, ethanol and glycerol are mixed together, a suitable amount of water is added thereto and then the active compound of the present invention is dissolved in the solution. Water is further added to the solution to a total volume of 100 ml to prepare a tonic.

Preparation 5

| | |
|---|---|
| 6-[4-(1-Cyclohexyl-1,2,3,4-tetrazol-5-yl)-butoxy]-3,4-dihydrocarbostyril<br>Isopropyl alcohol<br>Polyoxyethylene sorbitan monostearate<br>Water | 0.5 g<br>60.0 g (75 ml)<br>2.0 g<br>q.s. |
| Total volume | 100 ml |

Isopropyl alcohol and polyoxyethylene sorbitan monostearate are mixed together, a suitable amount of water is added thereto and then the active compound of the present invention is dissolved in the solution. Water is further added to the solution to a total volume of 100 ml to prepare a tonic.

Preparation 6

| | |
|---|---|
| 6-[4-(1-Cyclohexyl-1,2,3,4-tetrazol-5-yl)-butoxy]-3,4-dihydrocarbostyril<br>Ethanol<br>Glyceryl monocaprate<br>Water | 0.5 g<br>59.1 g (75 ml)<br>3.0 g<br>q.s. |
| Total volume | 100 ml |

Ethanol and glyceryl monocaprate are mixed together, a suitable amount of water is added thereto and the active compound of the present invention is dissolved in the solution. Water is further added to the solution to a total volume of 100 ml to prepare a tonic.

Pharmacological test

According to the method of Kubo et al. (Yakugaku Zasshi 108 (10) P971-978 (1988)), an experiment was conducted using six weeks old ddy male mice. Depilatory cream was applied to the back of mice, and after 8 minutes, wiped off with absorbent cotton soaked with hot water at 40 °C to remove hair at 4 to 5 $cm^2$. The next day, those mice with no injury and undepilated hair were selected and used as shaved mice. Each experimental group consisted of 15 mice.

Each 0.2 ml of a solution to be tested and a control solution were uniformly applied to the shaved part of mice with a brush once a day for 18 days.

Evaluation of the hair grow-promoting effect was conducted by evaluating a hair grow score at two day intervals for 18 days and testing a significance difference with Willcoxon Rank sum test. The following hair grow score standards were employed:

0: Completely no growth of hair is observed with naked eye
1: Recovery up to 25 % of normal state
2: Recovery up to 50 % of normal state
3: Recovery up to 75 % of normal state
4: Recovery up to nearly normal state

and each standard score were further subdivided into three stages and hair grow score was obtained for each mouse.

The samples used for testing for effect were as follows:

Control: 75 % etanol solution

Preparation of the present invention: 0.2 % 6-[4-(1-cyclohexyl-1,2,3,4-tetrazol-5-yl)butoxy]-3,4-dihydrocarbostyril in 75 % ethanol solution

The results are shown in Table 1.

Table 1

| | Term to be treated (day) | | | | | |
|---|---|---|---|---|---|---|
| Sample | 8 | 10 | 12 | 14 | 16 | 18 |
| Control | 0.03±0.03 | 0.07±0.04 | 0.18±0.09 | 0.78±0.17 | 1.23±0.21 | 1.72±0.20 |
| Prep. of invention | 0.12±0.07 | 0.17±0.07 | 0.72±0.17 | 1.45±0.23 | 2.25±0.27* | 2.58±0.28** |

Each value shows mean hair grow score ± S.E.

* $P<0.01$; ** $P<0.05$: significance difference against control group

## Claims

**Claims for the following Contracting States : CH, DE, DK, FR, GB, IT, LI, NL, SE**

1. Use of a carbostyril derivative having the general formula (1):

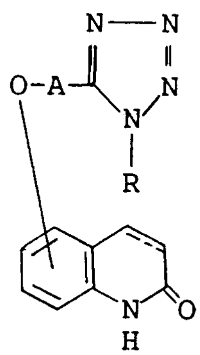

wherein A is a lower alkylene group, R is a cycloalkyl group, the bond between the 3-position and the 4-position of the carbostyril residue represents a single bond or a double bond, or a salt thereof for preparing a composition for hair-growing and hair-dressing.

2. The use of claim 1 wherein the carbostyril derivative is 6-[4-(1-cyclohexyl-1,2,3,4-tetrazol-5-yl)-butoxy]-3,4-dihydrocarbostyril or a salt thereof.

3. The use of claim 1 or 2 wherein the composition is a hair grow-promoting agent.

4. The use of claim 1 or 2 wherein the composition is in the form of a cosmetics for hair.

5. The use of claim 4 wherein the composition is in the form of a hair tonic.

**Claims for the following Contracting State : ES**

1. A method of use of a carbostyril derivative having the general formula (1):

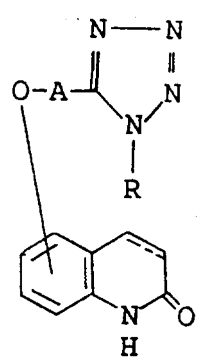

wherein A is a lower alkylene group, R is a cycloalkyl group, the bond between the 3-position and the 4-position of the carbostyril residue represents a single bond or a double bond, or a salt thereof for preparing a composition for hair-growing and hair-dressing.

2. The method of claim 1 wherein the carbostyril derivative is 6-[4-(1-cyclohexyl-1-2,3,4-tetrazol-5-yl)-butoxy]-3,4-dihydrocarbostyril or a salt thereof.

3. The method of claim 1 or 2 wherein the composition is a hair grow-promoting agent.

4. The method of claim 1 or 2 wherein the composition is in the form of a cosmetics for hair.

5. The method of claim 4 wherein the composition is in the form of a hair tonic.

**Patentansprüche**
**Patentansprüche für folgende Vertragsstaaten : CH, DE, DK, FR, GB, IT, LI, NL, SE**

1. Verwendung eines Carbostyril-Derivats der allgemeinen Formel (1)

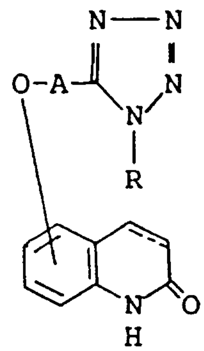

worin
A eine niedere Alkylen-Gruppe ist,
R eine Cycloalkyl-Gruppe ist,
die Bindung zwischen der 3-Position und der 4-Position des Carbostyril-Restes eine Einfach-Bindung oder eine Doppel-Bindung darstellt,
oder eines Salzes derselben zur Herstellung einer Zusammensetzung für den Haarwuchs und die Haarpflege.

2. Verwendung nach Anspruch 1, worin das Carbostyril-Derivat 6-[4-(1-Cyclohexyl-1,2,3,4-tetrazol-5-yl)-butoxy]-3,4-dihydrocarbostyril oder ein Salz desselben ist.

3. Verwendung nach Anspruch 1 oder 2, worin die Zusammensetzung ein den Haarwuchs förderndes Mittel ist.

4. Verwendung nach Anspruch 1 oder 2, worin die Zusammensetzung in Form eines Haarpflegemittels vorliegt.

5. Verwendung nach Anspruch 4, worin die Zusammensetzung in Form eines Haar-Tonikums vorliegt.

**Patentansprüche für folgenden Vertragsstaat : ES**

1. Verfahren zur Verwendung eines Carbostyril-Derivats der allgemeinen Formel (1)

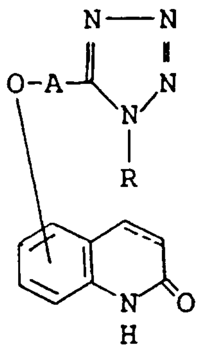

worin
A eine niedere Alkylen-Gruppe ist,
R eine Cycloalkyl-Gruppe ist,
die Bindung zwischen der 3-Position und der 4-Position des Carbostyril-Restes eine Einfach-Bindung oder eine Doppel-Bindung darstellt,
oder eines Salzes derselben zur Herstellung einer Zusammensetzung für den Haarwuchs und die Haarpflege.

2. Verfahren nach Anspruch 1, worin das Carbostyril-Derivat 6-[4-(1-Cyclohexyl-1,2,3,4-tetrazol-5-yl)-butoxy]-3,4-dihydrocarbostyril oder ein Salz desselben ist.

3. Verfahren nach Anspruch 1 oder 2, worin die Zusammensetzung ein den Haarwuchs förderndes Mittel ist.

4. Verfahren nach Anspruch 1 oder 2, worin die Zusammensetzung in Form eines Haarpflegemittels vorliegt.

5. Verfahren nach Anspruch 4, worin die Zusammensetzung in Form eines Haar-Tonikums vorliegt.

**Revendications**
**Revendications pour les Etats contractants suivants : CH, DE, DK, FR, GB, IT, LI, NL, SE**

1. Utilisation d'un dérivé de carbostyrile de formule générale (1) :

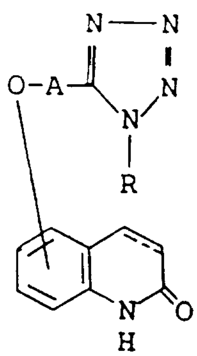

dans laquelle A est un groupe alkylène inférieur, R est un groupe cycloalkyle, la liaison entre les positions 3 et 4 du reste carbostyrile représente une liaison simple ou une liaison double, ou d'un de

ses sels, pour préparer une composition pour la pousse des cheveux et le coiffage des cheveux.

**2.** Utilisation selon la revendication 1, dans laquelle le dérivé de carbostyrile est le 6-[4-(1-cyclohexyl-1,2,3,4-tétrazole-5-yl)-butoxy]-3,4-dihydrocarbostyrile ou un de ses sels.

**3.** Utilisation selon la revendication 1 ou 2, dans laquelle la composition est un agent activateur de la pousse des cheveux.

**4.** Utilisation selon la revendication 1 ou 2, dans laquelle la composition est sous la forme d'un cosmétique capillaire.

**5.** Utilisation selon la revendication 4, dans laquelle la composition est sous la forme d'un tonique capillaire.

**Revendications pour l'Etat contractant suivant : ES**

**1.** Procédé d'utilisation d'un dérivé de carbostyrile de formule générale (1):

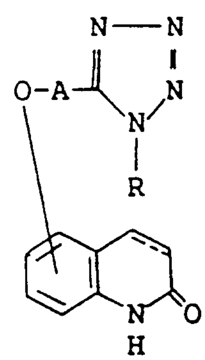

dans laquelle A est un groupe alkylène inférieur, R est un groupe cycloalkyle, la liaison entre les positions 3 et 4 du reste carbostyrile représente une liaison simple ou une liaison double, ou d'un de ses sels, pour préparer une composition pour la pousse des cheveux et le coiffage des cheveux.

**2.** Procédé selon la revendication 1, dans lequel le dérivé de carbostyrile est le 6-[4-(1-cyclohexyl-1,2,3,4-tétrazole-5-yl)-butoxy]-3,4-dihydrocarbostyrile ou un de ses sels.

**3.** Procédé selon la revendication 1 ou 2, dans lequel la composition est un agent activateur de la pousse des cheveux.

**4.** Procédé selon la revendication 1 ou 2, dans lequel la composition est sous la forme d'un cosmétique capillaire.

**5.** Procédé selon la revendication 4, dans lequel la composition est sous la forme d'un tonique capillaire.